# EUROPEAN PATENT SPECIFICATION

(11) **EP 1 309 862 B1**
(45) Date of publication and mention of the grant of the patent: **23.01.2008**
(21) Application number: 01972799.9
(22) Date of filing: 17.08.2001
(51) Int. Cl.: G01N 33/50

(54) **METHOD AND REAGENT FOR THE ANALYSIS OF BLOOD SAMPLES, IN PARTICULAR FOR VETERINARY APPLICATIONS**
VERFAHREN UND REAGENS ZUR ANALYSE VON BLUTPROBEN, IM BESONDEREN FÜR TIERÄRZTLICHE ANWENDUNGEN
PROCEDE ET REACTIF PERMETTANT D'EFFECTUER L'ANALYSE D'ECHANTILLONS SANGUINS, EN PARTICULIER POUR DES APPLICATIONS VETERINAIRES

(30) Priority: 17.08.2000 EP 00202889
(43) Date of publication of application: 14.05.2003
(73) Proprietor: Mallinckrodt Baker B.V., 7400 AA Deventer (NL)
(72) Inventor: KIEFTENBELD, Wilhelmus, Hermanus, Hendrikus, Maria, NL-8101 CV Raalte (NL)
(74) Representative: Jorritsma, Ruurd
(86) International application number: PCT/NL2001/000611
(87) International publication number: WO 2002/014861

(56) References cited:
- EP-A- 0 424 871
- EP-A- 0 444 241
- EP-A- 0 794 435
- WO-A-84/02777
- FR-A- 2 621 695
- GB-A- 2 279 653
- US-A- 4 185 964
- US-A- 4 745 071
- US-A- 5 763 280

## Description

The present invention relates to a method for the analysis of blood samples, and to reagents for use in such a method.

In particular, the invention relates to a method for the analysis of blood samples intended for veterinary applications, e.g. for the analysis of the blood of pets and/or other non-human mammals, and to reagents for use in such a method.

In one particularly preferred embodiment, the invention relates to a method for the analysis of blood samples obtained from cats, dogs and/or horses, and to reagents for use in such a method.

Methods for the analysis of human blood samples are well known in the art. Usually, in these known methods, the amounts of the erythrocytes (red blood cells or "RBCs"), of the thrombocytes (platelets or "PLTs") and/or of the leukocytes (white blood cells or "WBCs") in the blood sample are determined. In doing so, the leukocytes are usually further differentiated into three subpopulations, e.g. the lymphocytes, the so-called "mixed cell population" consisting mainly of monocytes, and the granulocytes, of which the latter may be even further differentiated in neutrophils, eosinophils and basophils. Abnormalities in the relative or absolute amounts of one or more of these types of blood cells may be helpful in the diagnosis of, or may even be indicative for, certain disease states and/or disorders.

Generally, in these known methods, the amount of each specific cell type is determined by counting the number of cells of said type that is present in the sample. This is usually carried out using automated cell counting equipment also referred to as "cell sorters" or "cell counters", which can differentiate between and so count the cells present in a sample, based upon differences in cell size.

As such cell sorters are well established in the art, they need not be discussed in detail here. However, for the purposes of the present disclosure, it should be noted that cell sorters may generally be distinguished in aperture impedance apparatus and light scatter apparatus, depending upon the specific technique used to measure/detect the individual cells as they pass through the cell sorter. Although both types of apparatus can be used to measure/detect all of the above cell types essentially simultaneously, cell sorters working on the basis of aperture impedance generally have the advantage that they are less expensive. Also, the invention disclosed herein is particularly intended for use with aperture impedance apparatus (although in its broadest sense, it is not limited thereto).

However, with neither type of cell sorter can the different types of blood cells in a sample be counted directly, i.e. in the blood sample as such. This is because the different cell types present in the sample are generally of about the same size, so that they interfere with the counting of each individual type. For instance, non-lysed erythrocytes may interfere with the counting of the leukocytes; also, the different sub-populations of the leukocytes may interfere with the counting of specific sub-population.

Thus, in all known methods for the analysis of human blood samples, it is necessary to pre-treat the blood sample so as to allow for differentiation between the different cell types during measurement/cell counting.

Generally, this is carried out in a two-step procedure, of which the first step comprises diluting the blood sample using an isotonic diluent. Thereafter, in a second step, a lysing agent is added to the diluted sample. This lysing agent removes the cytoplasm from the leukocytes - also referred to as "stripping" - which leads to differences in size between different leukocyte subpopulations, which can then each be detected/counted separately in the cell sorter. The lysing agent also lyses (e.g. stromatolyses) the red blood cells to release the hemoglobin therefrom, which may then be determined either as a cyanomethemoglobin complex (cyanide-based) or as a hematin-like complex (cyanide-free), depending upon whether a cyanide-based or a cyanide-free lysing agent is used.

In addition to the lysing step, part of the blood sample obtained after the first dilution step may also be diluted further and may then be used in a separate step to determine the amount of red blood cells, amount of blood platelets and/or to determine the mean corpuscular volume (MCV).

In the currently available cell sorters, the above dilution and lysis steps are also carried out automatically. Thus, the operator only needs to enter the blood sample into the cell counter and then collect the read out containing the results of the detection/cell counts.

To allow for a proper differentiation between the different cell types, the composition of the diluting agent and of the lysing agent should be carefully selected. In practice, most often a diluting agent and a lysing agent are used which are specifically intended/formulated for use with each other, and also for use with a specific type of cell sorter. Such diluents and lysing agents are also marketed as commercial preparations/formulations, for instance by applicant.

EP-A-044241 describes a cyanide-free reagent for counting the number of leukocytes and measuring the hemoglobin concentration in blood.

For a further description of the above methods for the analysis of human blood samples, and of diluents and lysing agents for use in such methods, reference is made to the prior art, such as US-A-4485175, US-A-4346018 and the further prior art mentioned therein.

Although the above techniques and reagents are now routinely used for the analysis of human blood samples, the art does not describe similar techniques for veterinary applications, e.g. for the analysis of the blood of animals. Thus, it is a first object of the invention to provide such a method, and in particular to provide such a method for the analysis of the blood of cats, dogs and other pets, as well as horses and other mammals of interest.

In particular, it is an object of the invention to provide such a method that can be carried out using cell sorters known per se for the analysis of human blood samples, and in particular using aperture impedance equipment known per se. This would have the advantage that any such method can be carried out using apparatus already (commercially) available, and therefore would not require any specifically designed equipment.

To achieve this object, in their research leading up to the present invention, the inventors have first tried to apply diluents and lysing agents known for the analysis of human blood to the analysis of the blood of animals. However, in doing so, the inventors found that these known diluents and lysing agents are in fact not suited for the analysis of the blood of animals.

In particular, it was found that, due to the differences between human blood and animal blood (i.e. the blood of non-human mammals), the diluents and lysing agents known for the analysis of human blood, when used in the analysis of animal blood, do not allow for sufficient differentiation into the different subpopulations of the leukocytes and may lead to to inadequate MCV and pletelet measurement. It was also found that the cells, and in particular the leukocytes, of animal blood are often much more vulnerable/sensitive than the cells of human blood and that this may lead to problems with cell stability during the detection/counting of the cells when diluents and/or lysing agents for the analysis of human blood are used.

A further problem that occurs in developing a method for the analysis of the blood of animals, and in developing diluents and lysing agents for use in such a method, is that the blood of a given species of animal (mammal) not only differs from the blood of humans, but also differs from the blood of other species of animals (mammals). For instance, the blood of cats, dogs and horses contain different hemoglobin structures, which may oxidize differently.

Thus, a diluent and a lysing agent that are suited for the analysis of the blood from one species of animal may not necessarily be (equally) suited for the analysis of the blood from another species. However, it would neither be practical nor economically feasible to provide a different diluent and/or a different lysing agent for each species of animal.

Thus, a specific object of the invention is to provide a method, as well as to provide a diluent and a lysing agent for use in such a method, that can equally be used to analyze the blood of cats, dogs and/or horses.

It has been found that by the use of the cyanide-free lysing agent of the invention, the different hemoglobin structures that may occur from animal species to animal species may be determined in an equally suitable manner.

Thus, with advantage, by the use of said diluent and lysing agent, the invention provides a method and the use of reagents for the analysis of non-human mammalian blood, which is equally suited for the analysis of the blood of different species of non-human mammals, and in particular of cats, dogs and horses, which allow for a differentiation of the leukocytes into at least two, and preferably three different subpopulations based upon differences in size, and which allow for the measurement of hemoglobin without the need to use of any cyanide-containing reagents.

Accordingly, the invention provide the use of a cyanide-free lysing agent, which means that cyanide is no longer required to analyse the hemoglobin species. Also, the use of said lysing agent with advantage provides for a 2- or 3-part differential analysis. This combination of features (i.e. a cyanide-free lysing agent and a 2- or 3-part analysis) has not yet been achieved in the art: the only lysing agents currently on the market which are capable of providing a 2- or 3-part differential analysis always contain cyanide.

The method and lysing agent used in the invention (further) differ from known methods and lysing agents (i.e. for the analysis of human blood) in the concentration of the quaternary ammonium compounds (vide below) in the final WBC/Hb dilution (vide again below), which is lower according to the invention (i.e. between 1.5 and 4.5 g/l for the invention compared to between 4.5 and 7.5 g/l for the analysis of human blood).

In addition, the performance of the lysing agent may be further improved by the use of further components, e.g. as outlined below.

Accordingly, in a first aspect, the invention relates to a lysing agent for use in the analysis of the blood and/or of a blood sample of a non-human mammal, said lysing agent comprising an aqueous solution, which
- is such that it provides, upon dilution to the final WBC/Hb dilution (i.e. the solution in which the WBCs and Hb are measured/counted), a pH of between 4.0 and 7.5 for said final WBC/Hb dilution; and which
- contains at least one quaternary ammonium compound, in a concentration such that, upon dilution to the final WBC/Hb dilution, the total concentration of quaternary ammonium compounds in said final WBC/Hb dilution is between 1.5 and 4.5 grams per liter;
and which optionally further contains one or more components for lysing agents known per se, such as those mentioned hereinbelow.

With respect to the pH, concentration of the ammonium compound(s) and the "final WBC/Hb dilution", it should be noted that the amount of lysing agent and/or the lyse volume added may differ, depending upon the specific instrument (i.e. the type of cell counter/cell sorter) used. For instance, some haematology analysers use a lyse addition of 0.7 ml, whereas other type use an addition of 2.0 ml.

Accordingly, the concentration of the quaternary ammonium compound(s) in the above lysing agent of the invention may also vary, i.e. depending upon the specific instrument used. Most preferably, the concentration of the quaternary ammonoum compound will be adapted for use with a specific instrument, i.e. such that, in the final WBC/Hb solution as provided by the instrument used (i.e. the dilution at which the WBCs and Hb are measured/counted, which is essentially the same for all currently used instruments), the total concentration of quaternary ammonium is between 1.5 and 4.5 g/l. Usually, for this purpose, the concentration of the quaternary ammonium compound(s) in the initial lysing agent will generally be between 2.0 and 30.0 g/l, depending upon the instument used.

As an example a list of instrument manufacturer's is given with the quaternary ammonium compound(s) concentration in the lysing:
- Medonic (model CA530): 3.0 - 6.0 g/L (final WBC dilution 1:400)
- Diatron (model Abacus): 26.0 - 29.0 g/L (final WBC dilution 1:186)
- ABX (model ABC VET Micros): 17.0 - 20.0 g/L (final WBC dilution (1:300)

Thus, generally, the skilled person will be able to suitably select/adapt the lysing agent, i.e. the pH/amount of acid(s); the concentration of ammonium compound(s) and the concentration(s) of the optional further components, i.e. based upon the disclosure herein and upon available information on the specific apparatus used (e.g. from the manual that accompanies the apparatus).

The invention also relates to the use of such a lysing agent in analyzing the blood or a blood sample of a non-human mammal. In particular, the invention relates to the use of such a lysing agent in the lysis of (the erythrocytes present in) a blood sample of a non-human mammal and/or in the "stripping" of (the leukocytes present in) a blood sample of a non-human mammal, i.e. as part of the analysis of said blood sample. More in particular, the invention relates to the use of such a lysing agent in the lysis and/or stripping of such a blood sample that has been diluted with a diluent as described above, i.e. as part of the analysis of said blood sample.

In a second aspect, the invention relates to the use of a diluent in the analysis of the blood and/or of a blood sample of a non-human mammal, said diluent comprising an aqueous solution:
- having an osmolality of between 290 and 400;
- having a pH of between 6.0 and 8.0;
- optionally at least one coagulant chosen from EDTA, citrate/citric acid and/or salicylic acid; or a combination thereof,
and optionally further containing one or more components for diluents known per se, such as those mentioned hereinbelow.

When EDTA is present in the diluent of the invention, it may be used in any form suitable for hemocytometry purposes, including but not limited to its di- and tripotassium salts and/or its di-, tri- and tetrasodium salt, or any combination thereof.

When citrate is present in the diluent of the invention, optionally in combination with citric acid, the citrate may be in any (salt) form suitable for cytohemometry purposes, including but not limited to trisodium citrate.

When salicylic acid is present in the diluent of the invention, it may used be in any form - including but not limited to equivalent forms - suitable for cytohemometry purposes, including but not limited to sulfosalicylic acid and/or a suitable salts form such as sodium salicylate.
Preferably, in addition to the at least one anti-coagulant chosen from EDTA, citrate/citric acid and/or salicylic acid (or combinations thereof), the diluent of the invention comprises at least one additional anti-coagulant (i.e. different from/further to EDTA, citrate/citric acid and/or salicylic acid or combinations thereof). This "additional anti-coagulant" may be any anti-coalgulant suitable for hemocytometry purposes known per se (or any suitable combination of two or more such anti-coagulants), and may in particular may be chosen so as to make the diluent (even) better suited for use with (i.e. adapt the diluent even bettet to) the lysing agent. For instance, said "additional anti-coagulant" may be chosen from:
- Oxalate, e.g. in the form of a salt suitable for cytohemometry purposes such as potassium oxalate;
- Fluoride, e.g. in a form suitable for cytohemometry purposes such as sodium fluoride);
or a suitable combination thereof.

Preferably, however, the use of heparin should be avoided, as it may be less suited for hemocytometry purposes.
The invention also relates to the use of such a diluent in analyzing the blood or a blood sample of a non-human mammal. In particular, the invention relates to the use of such a diluent in diluting a blood sample of a non-human mammal, i.e. as part of the analysis of said blood sample.

In a further aspect, the invention relates to a method for the analysis of the blood and/or of a blood sample of a non-human mammal, said method comprising the steps of:
a) providing a blood sample of said non-human mammal;
b) diluting said blood sample with a diluent as described hereinabove;
c) lysing/stripping the diluted blood sample of step b) with a lysing agent as described hereinabove;
d) optionally counting, in the lysed/stripped blood sample obtained in step c), the leukocytes or at least one subpopulation thereof, and/or determining, in the lysed/stripped blood sample obtained in step c), the hemoglobin content (e.g. as a hematin-complex as described hereinbelow).

In this method, the sample in/of which in step d) the leukocytes and/or the hemoglobin content is counted/determined preferably:
- has a pH of between 4.0 and 7.5 and for said final WBC/Hb dilution; and
- contains at least one quaternary ammonium compound in a total concentration of between 1.5 and 4.5 grams per liter;

The invention will now be discussed in more detail hereinbelow with reference to the steps a) to d) of this method. It should however be noted that these steps will usually be carried out automatically by the cell sorter used.

The blood or blood sample used in step a) may have been obtained from any animal, and in particular from any non-human mammal, and more in particular from a mammalian pet (including but not limited to cats, dogs and horses) and/or from an agronomically important mammal (including but not limited to sheep, cow/cattle, goat, lamb, pig, etc.).

The invention is particularly suited for the analysis of blood or a blood sample of a cat, dog or horse.

Preferably, the osmolality of said animal blood sample is between about 290 and 325 mOsmol/kg. In particular, the blood or blood sample may have been obtained from a cat (osmolality of between 310-320 mOsmol/kg), a dog (osmolality of between 295-320 mOsmol/kg) or a horse (osmolality of about 320 mOsmol/kg).

By comparison, the osmolality of human blood is about 275-300 mOsmol/kg. Although the invention is not limited to any specific mechanism or explanation, it may be that this difference in osmolalilty between human blood and animal blood - which for instance means that the erythrocytes in animal blood are much smaller than the erythrocytes in human blood - may be one of the reasons why diluents known for the analysis of human blood are less suitable for the analysis of the blood of animals.

The blood sample may be obtained in any suitable manner known per se, such as by venous blood. Blood from cats and dogs may for instance be drawn either from the neck or paw vein. From horses almost always the neck vein will be used to draw blood. The blood sample may also be obtained as a sample containing EDTA or another suitable anti-coagulants, e.g. using a collection tube containing such an anti-coagulant.

Preferably, the blood sample is analyzed no later than 24 hours after collection, and preferably about 20-30 minutes after collection. Usually, the sample to be analyzed will have a volume of between 0.5 ml and 5 ml, depending upon the concentration of the anti-coagulant and the cell sorter used.

In step b), the blood sample is diluted with the diluent of the invention. This diluent generally comprises an aqueous solution with an osmolality of between 290 and 400 mOsmol/kg, preferably between 340 and 400 mOsmol/kg, in particular between 350 and 390 mOsmol/kg, and more in particular between 365 and 375 mOsmol/kg. By comparison, diluents for the analysis of human blood usually have an osmolality of between 240 and 350.

The osmolality of the diluent used in the invention is specifically adapted to the analysis of non-human mammalian blood, and even more specifically to the analysis of the blood of cats, dogs and/or horses. More generally, the osmolality may be chosen such that the hematocrit value (e.g. the ratio of cells to the total blood volume) as determined with the techniques described herein using the diluent of the invention differs by no more than 2% from the hematocrit value as determined for essentially the same sample (e.g. from the same animal or from the same species of animal) by microcentrifugation (a reference technique).

The diluent used in the invention is also slightly acidic, with a pH that is preferably between 6.0 and 8.0, preferably between 6.0 and 7.0, and in particular between 6.4 and 6.6. By comparison, diluents for the analysis of human blood usually have a pH of between 6.5 and 7.5.

For instance, taking into account the above, a particularly preferred diluent of the invention may have an osmolality of between 365 and 375 mOsmol/kg and pH of between 6.4 and 6.6.

The diluent used in the invention is further such that it provides for the presence of at least one (additional) anti-coagulant, i.e. in addition to EDTA which may (also) be present in the diluent, or which may already be present in the blood sample as collected, e.g. when an EDTA-anti-coagulated blood sample is used.

The presence of said "additional" anticoagulants may help to reduce/prevent the formation of thrombocyte aggregates, which may be associated with/caused by the presence of increased levels of adrenalin in the blood sample, e.g. due to the stress the animal may be under when the blood sample is collected. Such increased levels of adrenalin in the sample may in turn lead to the activation of clotting factors such as Factor VIII, which may prime or trigger the blood clotting cascade, thus leading to the formation of platelet aggregates. As such aggregates are difficult to separate, they may lead to inaccuracies in the determination of the blood platelet count. Also, they may interfere with the differentiation/counting of the leukocytes, and in particular with the differentiation/counting of the lymphocytes. The aforementioned is in particular a problem when blood samples are used that have been obtained from animals which are easily stressed, such as cats.

Suitable anti-coagulants include EDTA, citrate/citric acid and salicylic acid, or a combination thereof. These may be used in a conventional amounts known per se, for instance:
- EDTA: between 0.001 and 0.4 %, in particular approx. 0.04%;
- Citrate/citric acid: between 0.01 and 1%, in particular approx. 0.25%;
- Salicylic acid: between 0.0001 and 0.1 %, in particular less than 0.01%

The diluent may further contain one or more further components for diluents known per se, which may again be present in amounts known per se. These may include, but are not limited to, the following components:
- Inorganic salts used for buffering the pH, such as phosphates (Na₂HPO₄, NaH₂PO₄ or KH₂PO₄), Borates (Na₂B₄O₇) and Tris(hydroxymethyl)aminomethane
- Urea compound, such as Dimethylolurea;
- Amino compounds, such as Procaine Hydrochloride;
- Inorganic sulphate salts, such as Na₂SO₄;
- Inorganic chloride salts, such as NaCl;
- Preservative, such sodium azide, monophenylglycol, sodium benzoate, thimerosal or sodium-1-hydroxypyridine-2-thione,
or any suitable combination thereof, which may.

A particularly preferred diluent used in the invention is an aqueous solution with an osmolality of between 340 and 400, and in particular about 370 mOsmol/ml; and a pH of between 6.0 and 7.0, and in particular about 6.5, which contains the following components (e.g. chosen from the specific compounds disclosed herein) per 1 liter of water:
- at least one inorganic salt: 13 - 21 g of which:
   - inorganic salt used for buffering: 2-8 g
   - inorganic sulphate salt: 9 - 10 g
   - inorganic chloride salt: 2 - 3 g
- one or more urea compounds: 1 - 3 g
- one or more amino compounds: 0.05 - 0.15 g
- anti-coagulants (total): < 3.0 g
- preservatives (total) < 1.0 g
For instance, a diluent of the invention may comprise per 1 liter of water:
- Na₂HPO₄ 3-4g
- NaH₂PO₄.1H₂O 2 - 3g
- Dimethylolurea 1-3g
- Procaine Hydrochloride 0.05 - 0.15 g
- Na₂SO₄ 9-10g
- NaCl 2 - 3g
- Citric acid 0.5 - 1.0 g
- Sodium citrate 1 - 2 g
- Sulfosalicylic acid 0.004 - 0.008 g
- Sodium EDTA 0.3 - 0.4 g
- Preservative 0.05 - 0.10 g

Using said diluent, the blood sample is diluted to a dilution of between 1:100 and 1:200. Thereupon, in step c), the diluted blood sample of step b) is lysed/stripped using the lysing agent of the invention, as further described below.

In addition to said lysis step c), part of the diluted blood sample of step b) may be collected and used in a separate step for the determining *inter alia* the red blood cell count, the blood platelet count and/or the mean corpuscular volume (MCV). Usually, for this purpose, part of the diluted blood sample of step b) is further diluted, e.g. to a final dilution of between 1:10.000 and 1:60.000, e.g. using (preferably) the same diluent as used in step b) or another suitable diluent. Thereupon, one or more of the aforementioned parameters of the blood sample may be determined, e.g. in a manner known per se for the analysis of human blood. For instance, the red blood cell count and the platelet count may be determined using an aperture impedance technique, for instance at aperture sizes of between 60 and 100 µm (micrometer).

The lysing agent used in the invention is generally an aqueous solution that contains at least one (preferably water-soluble) quaternary ammonium compound, such that after addition of the lysing reagent the total concentration of quaternary ammonium compounds in the final WBC/Hb dilution is between 1.5 and 4.5 grams per liter.

The quaternary ammonium compound may be any quaternary ammonium compound or combination of quaternary ammonium compounds known per se for lysing agents, such as a dodecyltrimethylammoniumhalogenide, a tetradecyltimethyl-ammoniumhalogenide, a hexadecyltrimethyl- ammoniumhalogenide and/or a ethylhexadecyldimethylammoniumhalogenide (in which the term "halogenide" refers to the presence of a halogen anion such as fluoride, chloride, bromide or iodide as the counterion) and/or the quaternary ammonium compounds mentioned in US-A-4346018 and/or US-A-4485175, or any combination thereof.

Particularly preferred are quaternary ammonium compounds of the general formula:

[(R₁)₃N⁺-(CH₂)ₙCH₃] X⁻

in which:
- each group R₁ is a independantly a C₁-C₃ alkyl group, preferably a methyl or ethyl group, and most preferably a methyl group;
- n is an integer of between 5 and 21, preferably between 7 and 15, more preferably between 9 and 13, and in particular 11; and
- X⁻ is a suitable anion, such as chloride, bromide or fluoride;
of which particularly preferred examples are dodecyltrimethylammoniumbromide and dodecyltrimethylammoniumchloride.

The quaternary ammonium compounds, optionally together with one or more of the other components of the lysing agent as mentioned below, provide for the (stromato)lysis of the erythrocytes and the stripping of the leukocytes, so as to allow differentiation of the leukocyte-subpopulations. Thus, more generally, the concentration of the quaternary ammonium compound(s) in the lysing agent of the invention should be such that it allows for the lysis of the erythrocytes and for the stripping of the leukocytes, more preferably so as to allow differentiation of the leukocytes in at least any two, and preferably all three, of the leukocyte subpopulations mentioned above.

In this respect, it should be noted that the concentration of the quaternary ammonium compounds in the lysing agents used in the invention is generally lower than the concentrations of such quaternary ammonium compounds in lysing agents for the analysis of human blood samples. Usually, the concentrations of such quatenary ammonium compounds will be such that they provide, in the final WBC/Hb dilution as described above, a concentration of quaternary ammonium compounds between 4.5 - 7.5 grams per liter. By comparison, in the final WBC/Hb dilution for the analysis of human blood, the concentration of said quaternary ammonium compounds is usually between 4.5 and 7.5 g/l.

The lysing agents used in the invention also will be such that they can provide the final WBC/Hb dilution with a pH of between 4.0 and 7.5, preferably between 5.0 and 7.0, which is lower than the usual pH of lysing agents for the analysis of human blood, which usually have a pH of between 6.0 and 8.0 (and in particular of > 7.0 due to the presence of cyanide in most conventional lysing agents for 2- or 3- part differential analysis). The use of such a low(er) pH in the lysing agents of the invention (further) reduces/prevents the formation of platelet aggregates and facilitates the stromatolysis of the erythrocytes. Also, the lower pH of the lysing agents of the invention helps to stabilize the leukocytes.

The lysing agent used in the invention is preferably essentially cyanide-free, in that no cyanide-containing salts or other components for the analysis of hemoglobin (e.g. as a cyanide-containing complex) have been added. Instead, using the lysing agent of the invention, the hemoglobin is preferably converted into a cyanide-free hematin complex by the presence in the lysing agent of a suitable (an)ion, such as a chloride and/or bromide ion. This cyanide-free hematin-complex may then be measured (spectro)photometrically, as further described hereinbelow.

The lysing agent used in the invention may further contain one or more components for lysing agents known per se, in amounts known per se. These may include components such as:
- an suitable organic or an inorganic acid, for instance formic acid, acetic acid, butyric acid, citric acid, salicylic acid, phthalic acid or hydrochloric acid, or any suitable combination thereof. The presence of these acids may (further) contribute to the stromatolysis of the erythrocytes;
- a water soluble sulfate salt, such as sodium sulfate, potassium sulfate and/or ammonium sulfate, or any suitable combination thereof. These sulfates help to stabilize the leukocytes and/or to improve the differentiation of the leukocyte subpopulations;
- a glycol, such as monoethyleneglycol, diethyleenglycol, propyleneglycol an/or glycerol, or any suitable combination thereof; which may also help to stabilize the leukocytes and/or to improve the differentiation of the leukocyte subpopulations;
- an alcohol or alkoxyalcohol, such as methanol, ethanol, 1-propanol, 2-propanol, phenoxyethanol and/or 2-butoxyethanol, or any suitable combination thereof;
- a tertiary ammonium compound, and in particular a tertiary ammmonium oxide such as N,N-dimethyldodecylamine-N-oxide, which may (further) contribute to the stromatolysis of the erythrocytes and/or may help to stabilize the leukocytes and/or the hematin-complex.
   Such compounds may for instance have the formula 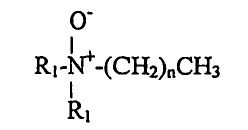
in which each group R₁ is a independently a C₁-C₃ alkyl group, preferably a methyl or ethyl group, and most preferably a methyl group; and in which *n* is an integer of between 5 and 21, preferably between 7 and 15, more preferably between 9 and 13, and in particular 11;
- A poly-oxy-ethylene-alkyl-phenyl-ether, such as Triton X-100, Nodidet P-40 or Tergitol, which may (further) contribute to the stromatolysis of the erythrocytes and/or may help to stabilze the leukocytes and/or hematin-complex.
   For instance, such a compound may for instance comprise an aromatic ring (at least) substituted (preferably in a para-configuration) with at least one (and preferably only one) lineair or (preferably) branched alkyl group with between 2 and 16, preferably between 4 and 12, more preferably between 6 and 10 carbon atoms, and in particular 8 carbon atoms such as a 1,1,3,3 tetramethylbutyl residue; and at least one (and preferably only one) substituted or (preferably) unsubstituted polyether-residue of the formula -O-(CH2-(CH2)ₚ-O)_{q}-H, in which *p* is 0 or an integer between 1 and 3 (and preferably 1) and q is an integer between 2 and 14 (and preferably 9).
or any suitable combination of two or more of these components.

A particularly preferred lysing agent used in the invention is an aqueous solution, which gives a pH in the final WBC/Hb dilution of between 4.0 and 7.5.

Even more in particular, the lysing agent may be such that it provides, in the final WBC/Hb dilution, the following components (e.g. chosen from the specific compounds mentioned herein) in the next concentrations, based upon 1 liter of final WBC/Hb dilution:
- one or more inorganic sulphate salts: 6 - 10 g
- one or more ammonium compounds: 6.5 - 13.5 g of which:
   - tertiary ammonium compounds: 5 - 9 g
   - quaternary ammonium compounds: 1.5 - 4.5 g
- one or more organic or inorganic acids: 0.2 - 1.0 ml
- one or more glycols: 6 - 10 ml
- one or more poly-oxy-alkylene-phenyl -alkyl ethers: < 1.0 ml

For example, the lysing agent may be such that it provides, in the final WBC/Hb dilution, the following compounds in the next concentrations, based upon 1 liter of final WBC/Hb dilution:
- Na₂SO₄ 6 - 10 g
- N,N-Dimethyl-dodecylamine-N-oxide 5 - 9 g
- Dodecyltrimethylammoniumbromide 1.5 - 4.5 g
- Formic acid 0.2 - 1.0 ml
- Ethylene glycol 6 - 10 ml
or alternatively:
- Na₂SO₄ 6-10g
- Dodecyltrimethylammoniumbromide 1.5 - 4.5 g
- Ethyldimethylhexadecylammoniumbromide < 0.5 g
- Triton X-100 < 0.5 g

The lysing agent is preferably added to the diluted blood sample of step b) in a ratio of between 0.25 and 2.50 ml, based upon the volume of the diluted blood sample obtained in step b). Generally, this will provide a final dilution, compared to the original blood sample, of between 1:200 and 1:500.

After the lysing agent has been added, the lysis/stripping is allowed to proceed for a suitable period of time, e.g. of between 2 and 15 seconds, at a suitable temperature, e.g. about 18 and 30 °C.

After the lysis step c), the leukocytes or at least one sub-population thereof are counted using a cell sorting/cell counting technique, preferably using an automated cell sorter or cell counter known per se for the analysis of human blood samples.

As mentioned above, the invention is in particular intended for use with cell sorters or cell counters that work according to the aperture impedance principle, such as the aperture impedance-based cell sorters marketed by Coulter, Abbott, Sysmex, Nihon Kohden, Erma, ABX, Medonic, Swelab, BioChem ImmunoSystems, Danam, Diatron, Melet, Medonic and Hycel. It should however be noted that the invention is its broadest sense encompasses the use of any type of cell sorter or cell counter, irrespective of its working principle, and thus also encompasses the use of cell sorters working according to (for instance) the light scattering principle.

The specific settings of the cell sorter used will depend upon the type of cell sorter used, on the specific diluent and lysing agent of the invention used, and on the blood sample to be analyzed. Suitable settings may easily be determined by skilled person, optionally after some simple initial experiments and/or a limited degree of trial and error. It is also envisaged that the diluents, lysing agents and/or kits of the invention may be marketed with a manual in which suitable settings for a given cell sorter and a given species of animal are mentioned.

Preferably, in step d), at least one, preferably at least two, and most preferably all three sub-populations of the leukocytes - i.e. the lymphocytes, the "mixed cell population" and the granulocytes - are measured/determined separately. Accordingly, the diluent and lysing agent used in the invention are most preferably such that they allow for the differentiation at least any two, preferably all three, of said leukocyte subpopulations. A suitable aperture size for the cell counter may be between 70 and 120 µm.

In addition to the measurement of the leukocytes or leukocyte-subpopulations, in step d), also the hemoglobin-content of the sample may be determined. For this purpose, the hemoglobin released from the lysed red blood cells during the lysis step is preferably converted, by the chloride and/or bromide ions in the lysing agent, to a cyanide-free, hematin-like complex, which can be measured/detected spectrophotometrically at a wavelength of between 500 nm and 600 nm, preferably about 535-545 nm. This can further be carried out in a manner known per se for human blood samples.

The invention will now be illustrated by means of the following non-limiting Experimental Part, as well as the Figures, in which:
- Figures 1A and 1B are print-outs showing the results of the analysis of blood of a cat using a Medtronic CA 530 analyser. Fig. 1A: results obtained using known reagents for the analysis of human blood (comparative); Fig. 1B: results obtained using the reagents of the invention.
- Figures 2A and 2B are print-outs showing the results of the analysis of blood of a dog using a Medtronic CA 530 analyser. Fig. 2A: results obtained using known reagents for the analysis of human blood (comparative); Fig. 2B: results obtained using the reagents of the invention.
- Figures 3A and 3B are print-outs showing the results of the analysis of blood of a horse using a Medtronic CA 530 analyser. Fig. 3A: results obtained using known reagents for the analysis of human blood (comparative); Fig. 3B: results obtained using reagents of the invention.

### Experimental Part:

In the Examples below, an isotonic diluent and lysing agent known per se for the analysis of human blood (collectively: "human reagents") and an isotonic diluent and lysing agent (collectively: "veterinary reagents") were used according to the invention. These had the following composition:

### Composition "human reagents":

### a. Isotonic diluent

| | Per liter |
|---|---|
| Na₂HPO₄ | 3,60 g |
| NaH₂PO₄.2H₂O | 2,18 g |
| Na₂SO₄ | 8,33 g |
| NaCl | 1,28 g |
| NaN₃ | 0,50 g |
| Tween 20 | 0,05 mL |
| NaF | 0,64 g |
| Na₂EDTA.2H₂O | 0,36 g |

### b. Lysing agent

| | Per liter |
|---|---|
| Na₂HPO₄ | 5.68 g |
| Na₂SO₄ | 8.33 g |
| NaCl | 4.75 g |
| Dodecyltrimethyl-ammoniumbromide | 4.00 g |
| Sodiumdodecylsulfaat | 0.10 g |
| Ethylhexadecyldimethyl-ammoniumbromide | 0.35 g |

### Composition "veterinary reagents":

### a. Isotonic diluent

| | Per liter |
|---|---|
| Na₂HPO₄ | 3,46 g |
| NaH₂PO₄. H₂O | 2,45 g |
| Dimethylolureum | 2,00 g |
| Procaine Hydrochloride | 0,11 g |
| Na₂SO4 | 9,72 g |
| NaCl | 2,38 g |
| Citroenzuur.1H₂O | 0,75 g |
| Natrium(III)citraat.2H₂O | 1,60 g |
| Sulfosalicylzuur | 0,05 g |
| NaN₃ | 0,10 g |
| Na₂EDTA.2H₂O | 0,36 g |

### b. Lysing agent

| | Per liter |
|---|---|
| Na₂SO₄ | 8.33 g |
| Dodecyltrimethyl-ammoniumbromide | 4.00 g |
| N,N-Dimethyl-dodecylamine-N-oxide | 0.65 g |
| Formic acid | 0.50 mL |
| Ethylene glycol | 8.00 mL |

### Example I: Analysis of the blood of a cat.

Two identical samples of blood from a cat were analysed on a Medonic CA530 analyzer.

One of the samples was analysed with the "veterinary reagents" mentioned below; the other one was analysed with the "human reagents" mentioned above as a comparison.

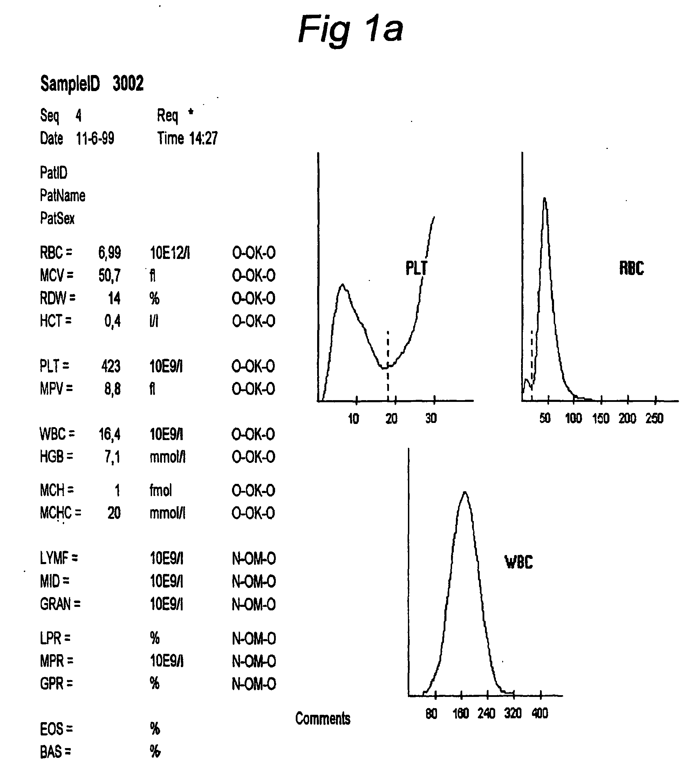

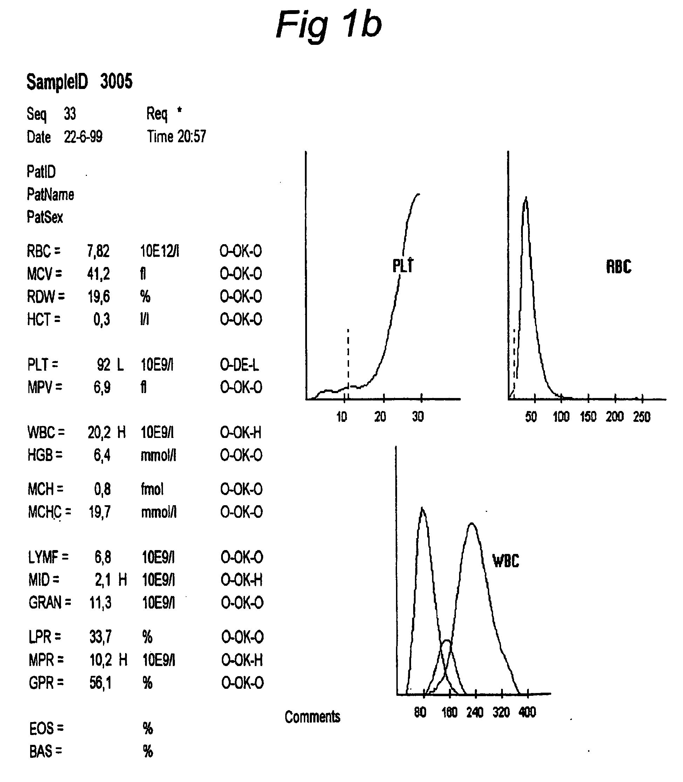

The results are given in Figure 1A ("human reagents", comparative) and Figure 1B ("veterinary reagents", invention), respectively.

As can be seen from these results, the results obtained using the veterinary reagents of were markedly more informative, *inter alia* allowing for a distinction of three different sub-populations (visible as three separate peaks) in the WBC count.

### Example II: Analysis of the blood of a dog.

Two identical samples of blood from a dog were analysed on a Medonic CA530 analyzer.

One of the samples was analysed with the "veterinary reagents" mentioned below; the other one was analysed with the "human reagents" mentioned above as a comparison.

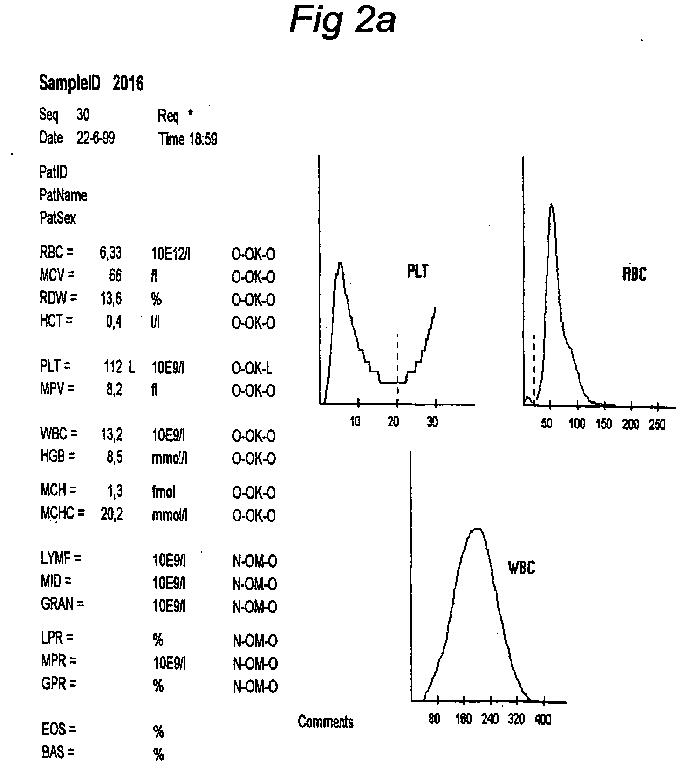

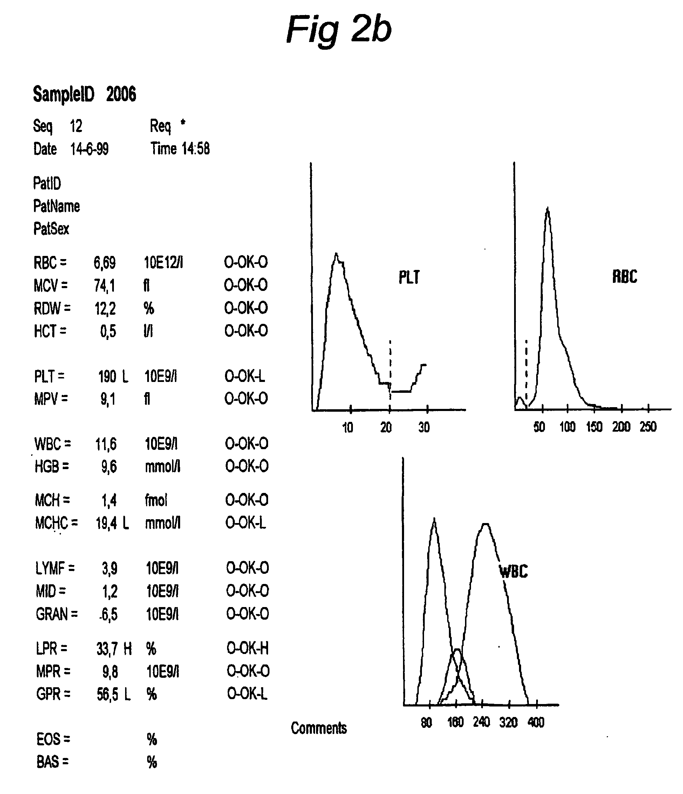

The results are given in Figure 2A ("human reagents", comparative) and Figure 2B ("veterinary reagents"), respectively.

As can be seen from these results, the results obtained using the veterinary reagents of the invention were markedly more informative, *inter alia* allowing for a distinction of three different sub-populations (visible as three separate peaks) in the WBC count.

### Example III: Analysis of the blood of a horse.

Two identical samples of blood from a horse were analysed on a Medonic CA530 analyzer.

One of the samples was analysed with the "veterinary reagents" mentioned below; the other one was analysed with the "human reagents" mentioned above as a comparison.

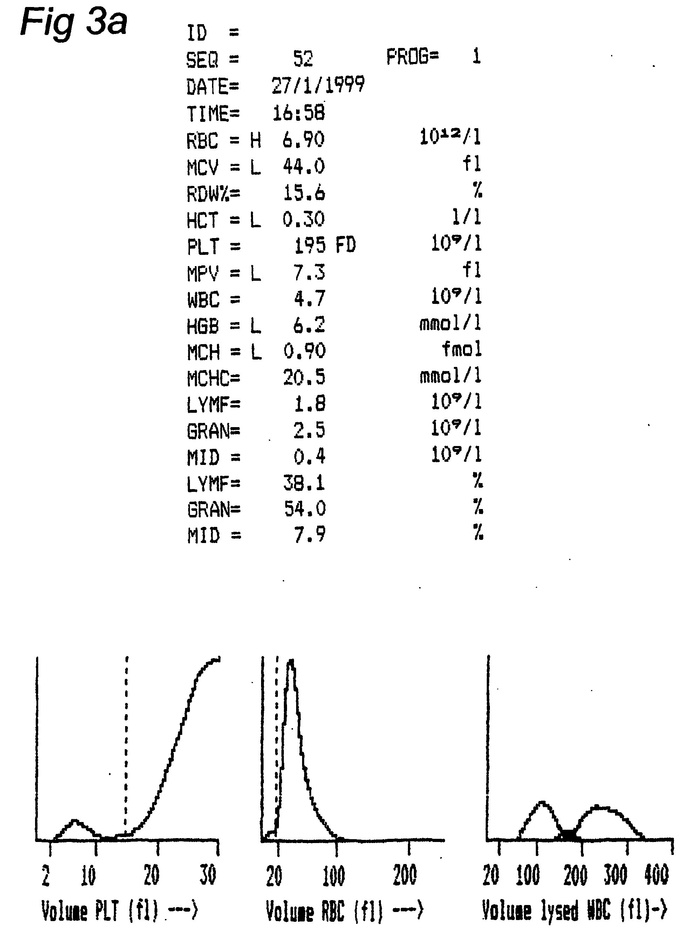

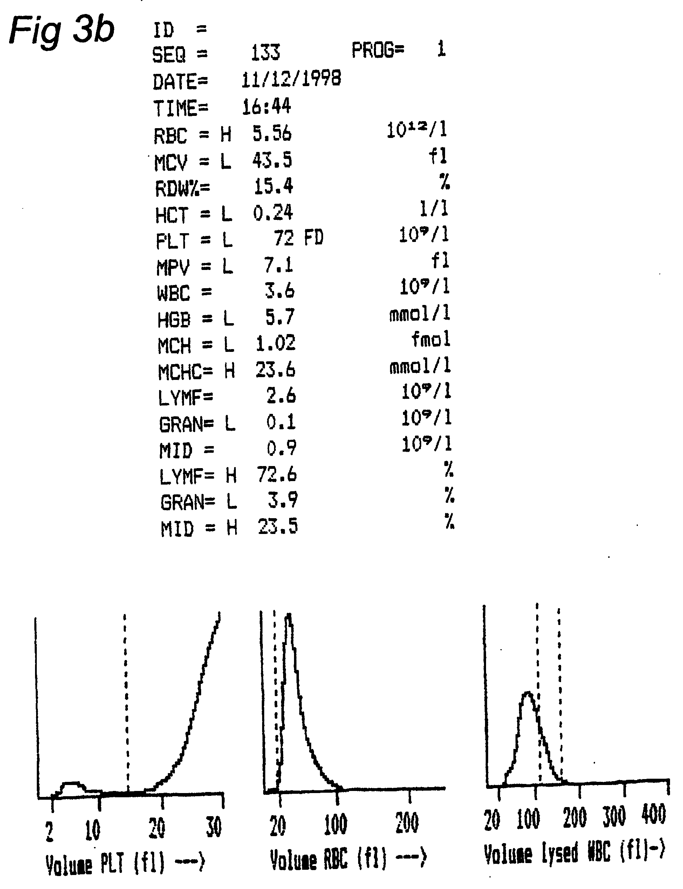

The results are given in Figure 3A ("human reagents", comparative) and Figure 3B ("veterinary reagents", invention), respectively.

As can be seen from these results, the results obtained using the veterinary reagents were markedly more informative, *inter alia* allowing for a distinction of different sub-populations in the WBC count.

## Claims

1. Use of a lysing agent in analyzing the blood or a blood sample of an a non-human mammal, and in the lysis of (the erythrocytes present in) a blood sample of a non-human mammal and/or in the "stripping" of (the leukocytes present in) a blood sample of a non-human mammal, i.e. as part of the analysis of said blood sample, wherein the lysing agent is essentially cyanide-free and provides, upon dilution to the final WBC/Hb dilution, a pH of between 4.0 and 7.5 and a total concentration of quaternary ammonium compounds in said final WBC/Hb dilution of between 1.5 and 4.5 grams per litre.

2. Use according to claim 1, wherein the lysing agent provides, in the final WBC/Hb dilution, the following components in the next concentrations, based upon 1 litre of final WBC/Hb dilution:
- one or more inorganic sulphate salts: 6-10 g
- one or more ammonium compounds: 6.5-13.5 g
of which:
- tertiary ammonium compounds: 5-9 g
- quaternary ammonium compounds: 1.5-4.5 g
- one or more organic or inorganic acids: 0.2-1.0 ml
- one or more glycols: 6-10 ml
- one or more poly-oxy-alkylene-phenyl-alkyl ethers: < 1.0 ml.

3. Use according to claim 1 or 2, in the lysis and/or stripping of a blood sample of a non-human mammal that has been diluted with a diluent comprising an aqueous solution:
- having an osmolality of between 290 and 400:
- having a pH of between 6.0 and 8.0:
- optionally at least one anti-coagulant chosen from EDTA, citrate/citric acid and/or salicylic acid: or a combination thereof;
and optionally further containing one or more components for diluents known per se

4. Use according to claim 3, wherein the diluent comprises at least one further anti-coagulant other than EDTA, citrate/citric acid and/or salicylic acid.

5. Use according to claim 3 or 4, wherein the diluent has an osmolalilty of between340 and 400mOsmol/kg, and a pH of between 6.0 and 7.0.

6. Use according to any of claims 3-5, wherein the diluent has an osmolality of between 365 and 375 mOsmol/kg and a pH of between 6.4 and 6.6.

7. Use according to any of claims 3-6, wherein the diluent comprises the following components per 1 litre of water:
- at least one inorganic salt: 13-21 g
of which:
- inorganic salt used for buffering: 2-8 g
- inorganic sulphate salt: 9-10 g
- inorganic chloride salt: 2-3 g
- one or more urea compounds: 1-3 g
- one or more amino compounds: 0.05-0.15 g
- anti-coagulant: < 3.0 g
- preservative : < 1. 0 g

8. Method for the analysis of the blood and/or of a blood sample of a non-human mammal, said method comprising the steps of:
a) providing a blood sample of said non-human mammal;
b) diluting said blood sample with a diluent comprising an aqueous solution:
- having an osmolality of between 290 and 400:
- having a pH of between 6.0 and 8.0;
- optionally at least one anticoagulant chosen from EDTA, citrate/citric acid and/or salicylic acid; or a combination thereof;
and optionally further containing one or more components for diluents known per se
c) lysing/stripping the diluted blood sample of step b) with a lysing agent that is essentially cyanide-free and that provides, upon dilution to the final WBC/Hb dilution, a pH of between 4.0 and 7.5 and a total concentration of quaternary ammonium compounds in said final WBC/Hb dilution of between 1.5 and 4.5 grams per litre
d) optionally counting, in the lysed/stripped blood sample obtained in step c), the leukocytes or at least one subpopulation thereof, and/or determining, in the lysed/stripped blood sample obtained in step c), the hemoglobin content.

9. Method according to claim 8, in which the sample in/of which in step d) the leukocytes and/or the hemoglobin content is counted/determined:
- has a pH of between 4.0 and 7.5 and for said final WBC/Hb dilution; and
- contains at least one quaternary ammonium compound in a total concentration of between 1.5 and 4.5 grams per litre.

10. Method according to claim 8 and/or 9, in which, in step d), at least two, and preferably all three of the leukocyte subpopulations comprised of the lymphocytes, the "mixed cell population" and/or the granulocytes are separately counted.

11. Method according to any of claims 8-10, in which part of the diluted blood sample obtained in step b) is further diluted, upon which, in said further diluted blood sample, the red blood cell count, the platelet count and/or the mean corpuscular volume is determined.

12. Use according to claim 1 or 3, or method according to any of claims 8-11, in which the non-human mammal is chosen from mammalian pets, including but not limited to cats, dogs and horses; and/or from agronomically important mammals, including but not limited to sheep, cows/cattle, goats, lambs and/or pigs.

13. Use according to claim 1 or 3, or method according to any of claims 8-11, in which the non-human mammal is chosen from a cat, a dog and/or a horse.

14. Use according to claim 1 or 3, use according to claim 12 or 13, or method according to any of claims 8-11, in which the analysis of the blood sample is by an aperture impedance technique.

## Patentansprüche

1. Verwendung eines Lysierungsagens beim Analysieren des Bluts oder einer Blutprobe eines nicht-menschlichen Säugers und bei der Lyse von (den Erythrozyten, welche vorhanden sind in) einer Blutprobe eines nicht-menschlichen Säugers und/oder beim "Strippen" von (den Leukozyten, welche vorhanden sind in) einer Blutprobe eines nicht-menschlichen Säugers, d. h. als Teil der Analyse von dieser Blutprobe, wobei das Lysierungsagens im Wesentlichen zyanidfrei ist und nach Verdünnung auf die endgültige WBC/Hb-Verdünnung einen pH von zwischen 4,0 und 7,5 und eine Gesamtkonzentration von quaternären Ammoniumverbindungen in der endgültigen WBC/Hb-Verdünnung von zwischen 1,5 und 4,5 Gramm pro Liter bereitstellt.

2. Verwendung nach Anspruch 1, wobei das Lysierungsagens in der endgültigen WBC/Hb-Verdünnung die folgenden Bestandteile in den nachfolgenden Konzentrationen, basierend auf einem Liter endgültiger WBC/Hb-Verdünnung, bereitstellt:
- ein oder mehrere anorganische Sulfatsalze: 6-10 g
- eine oder mehrere Ammoniumverbindungen: 6,5-13,5 g
davon:
- tertiäre Ammoniumverbindungen: 5-9 g
- quaternäre Ammoniumverbindungen: 1,5-4,5 g
- eine oder mehrere organische oder anorganische Säuren: 0,2-1,0 ml
- ein oder mehrere Glykole: 6-10 ml
- ein oder mehrere Polyoxyalkylen-Phenyl-Alkyl Ether: < 1,0 ml

3. Verwendung nach Anspruch 1 oder 2 bei der Lyse und/oder dem Strippen einer Blutprobe eines nicht-menschlichen Säugers, welche verdünnt worden ist mit einem Verdünnungsmittel, umfassend eine wässrige Lösung:
- welche eine Osmolalität von zwischen 290 und 400 aufweist,
- welche einen pH von zwischen 6,0 und 8,0 aufweist,
- optional zumindest ein Anti-Koagulans, ausgewählt aus EDTA, Citrat/Zitronensäure und/oder Salicylsäure, oder eine Kombination davon,
und optional weiterhin einen oder mehrere per se bekannte Bestandteile für Verdünnungsmittel enthält.

4. Verwendung nach Anspruch 3, wobei das Verdünnungsmittel mindestens ein weiteres Anti-Koagulans, welches nicht EDTA, Citrat/Zitronensäure und/oder Salicylsäure ist, umfasst.

5. Verwendung nach Anspruch 3 oder 4, wobei das Vedünnungsmittel eine Osmolalität von zwischen 340 und 400 mOsmol/kg und einen pH von zwischen 6,0 und 7,0 aufweist.

6. Verwendung nach einem der Ansprüche 3 bis 5, wobei das Verdünnungsmittel eine Osmolalität von zwischen 365 und 375 mOsmol/kg und einen pH von zwischen 6,4 und 6,6 aufweist.

7. Verwendung nach einem der Ansprüche 3 bis 6, wobei das Verdünnungsmittel die folgenden Bestandteile pro Liter Wasser umfasst:
- mindestens ein anorganisches Salz: 13-21 g
davon:
- zur Pufferung verwendetes anorganisches Salz: 2-8 g
- anorganisches Sulfatsalz: 9-10 g
- anorganisches Chloridsalz: 2-3 g
- eine oder mehrere Harnstoffverbindungen: 1-3 g
- eine oder mehrere Amminoverbindungen: 0,05-0,15 g
- Anti-Koagulans: < 3,0 g
- Konservierungsmittel: < 1,0 g.

8. Verfahren zur Analyse des Bluts und/oder von einer Blutprobe eines nicht-menschlichen Säugers, wobei das Verfahren die Schritte umfasst:
a) Bereitstellen einer Blutprobe von diesem nicht-menschlichen Säuger;
b) Verdünnen dieser Blutprobe mit einem Verdünnungsmittel umfassend eine wässrige Lösung:
- welche eine Osmolalität von zwischen 290 und 400 aufweist,
- einen pH von zwischen 6,0 und 8,0 aufweist,
- optional mindestens ein Anti-Koagulans, ausgewählt aus EDTA, Citrat/Zitronensäure und/oder Salicylsäure oder einer Kombination davon,
und optional weiterhin eine oder mehrere per se bekannte Bestandteile für Verdünnungsmittel enthält,
c) Lysieren/Strippen der verdünnten Blutprobe von Schritt b) mit einem Lysierungsagens, welches im Wesentlichen zyanidfrei ist und welches nach Verdünnung auf die endgültige WBC/Hb-Verdünnung einen pH von zwischen 4,0 und 7,5 und eine Gesamtkonzentration von quaternären Ammoniumverbindungen in der endgültigen WBC/Hb-Verdünnung von zwischen 1,5 und 4,5 Gramm pro Liter bereitstellt,
d) optional Zählen der Leukozyten oder zumindest einer Subpopulation davon in der lysierten/gestrippten in Schritt c) erhaltenen Blutprobe und/oder Bestimmen des Hämoglobingehalts in der lysierten/gestrippten in Schritt c) erhaltenen Blutprobe.

9. Verfahren nach Anspruch 8, bei welchem die Probe, in/von welcher in Schritt d) die Leukozyten und/oder der Hämoglobingehalt gezählt/bestimmt worden ist,
- einen pH von zwischen 4,0 und 7,5 und für die endgültige WBC/Hb-Verdünnung aufweist und
- mindestens eine quaternäre Ammoniumverbindung in einer Gesamtkonzentration von zwischen 1,5 und 4,5 Gramm pro Liter enthält.

10. Verfahren nach Anspruch 8 und/oder 9, bei welchem, bei Schritt d) zumindest zwei, und vorzugsweise alle drei der Leukozyten-Subpopulationen, umfassend die Lymphozyten, die "gemischte Zellpopulation" und/oder die Granulozyten, getrennt gezählt werden.

11. Verfahren nach einem der Ansprüche 8 bis 10, bei welchem ein Teil der in Schritt b) erhaltenen verdünnten Blutprobe weiter verdünnt wird, woraufhin in dieser weiter verdünnten Blutprobe die Zahl roter Blutzellen, die Blättchenzahl und/oder das mittlere Korpuskularvolumen bestimmt wird.

12. Verwendung nach Anspruch 1 oder 3 oder Verfahren nach einem der Ansprüche 8 bis 11, bei welcher/welchem der nichtmenschliche Säuger ausgewählt ist aus Säugetier-Haustieren, einschließlich aber nicht beschränkt auf Katzen, Hunde und Pferde und/oder aus agronomisch wichtigen Säugern, einschließlich aber nicht beschränkt auf Schaf, Kühe/Rind, Ziegen, Lämmer und/oder Schweine.

13. Verwendung nach Anspruch 1 oder 3 oder Verfahren nach einem der Ansprüche 8 bis 11, bei welcher/welchem der nichtmenschliche Säuger ausgewählt ist aus einer Katze, einem Hund und/oder einem Pferd.

14. Verwendung nach Anspruch 1 oder 3, Verwendung nach Anspruch 12 oder 13 oder Verfahren nach einem der Ansprüche 8 bis 11, bei welcher/welchem die Analyse der Blutprobe durch eine Öffnung-Impedanz-Technik erfolgt.

## Revendications

1. Utilisation d'un agent de lyse dans l'analyse du sang ou d'un échantillon sanguin d'un mammifère non humain, et dans la lyse des (érythrocytes présents dans) un échantillon sanguin d'un mammifère non humain et/ou dans le « dégarnissage » des (leucocytes présents dans) un échantillon sanguin d'un mammifère non humain, c'est-à-dire comme une partie de l'analyse dudit échantillon sanguin, dans laquelle l'agent de lyse est essentiellement exempt de cyanure et fournit, après la dilution à la dilution de WBC/Hb finale, un pH entre 4,0 et 7,5 et une concentration totale des composés d'ammonium quaternaire dans ladite dilution de WBC/Hb finale entre 1,5 et 4,5 grammes par litre.

2. Utilisation selon la revendication 1, dans laquelle l'agent de lyse fournit, dans la dilution de WBC/Hb finale, les composants suivants aux concentrations suivantes, par rapport à une dilution de WBC/Hb finale de 1 litre :
- un ou plusieurs sels de sulfate inorganiques : 6 - 10 g
- un ou plusieurs composés d'ammonium : 6,5 13,5 g
dont :
- des composés d'ammonium ternaire : 5 - 9 g
- des composés d'ammonium quaternaire : 1,5 -4,5 g
- un ou plusieurs acides organiques ou inorganiques : 0,2 - 1,0
- un ou plusieurs glycols : ml
- un ou plusieurs éthers de poly-oxy-alkylène-phényl-alkyle 6 - 10 ml
< 1,0 ml

3. Utilisation selon la revendication 1 ou 2, dans la lyse et/ou le dégarnissage d'un échantillon sanguin d'un mammifère non humain qui a été dilué ave un diluant comprenant une solution aqueuse :
- ayant une osmolalité entre 290 et 400 ;
- ayant un pH entre 6,0 et 8,0 ;
- éventuellement au moins un anti-coagulant choisi parmi l'EDTA, le citrate/acide citrique et/ou l'acide salicylique ; ou une combinaison de ceux-ci ;
et contenant en outre éventuellement un ou plusieurs composants pour le diluant connus par eux-mêmes.

4. Utilisation selon la revendication 3, dans laquelle le diluant comprend au moins un autre anti-coagulant différent de l'EDTA, du citrate/acide citrique et/ou de l'acide salicylique.

5. Utilisation selon la revendication 3 ou 4, dans laquelle le diluant a une osmolalité entre 340 et 400 mOsmoles/kg, et un pH entre 6,0 et 7,0.

6. Utilisation selon l'une quelconque des revendications 3 à 5, dans laquelle le diluant a une osmolalité entre 365 et 375 mOsmoles/kg et un pH entre 6,4 et 6,6.

7. Utilisation selon l'une quelconque des revendications 3 à 6, dans laquelle le diluant comprend les composants suivants par litre d'eau :
- au moins un sel inorganique : 13 - 21 g
dont :
un sel inorganique utilisé pour le tamponnage : 2 - 8 g
- un sel de sulfate inorganique : 9 - 10 g
- un sel de chlorure inorganique : 2 - 3g
- un ou plusieurs composés d'urée : 1 - 3 g
- un ou plusieurs composés aminés : 0,05 - 0,15 g
- un anti-coagulant : < 3,0 g
- un conservateur : < 1,0 g

8. Procédé d'analyse du sang et/ou d'un échantillon sanguin d'un mammifère non humain, ledit procédé comprenant les étapes consistant à :
a) fournir un échantillon sanguin dudit mammifère non humain;
b) diluer ledit échantillon sanguin avec un diluant comprenant une solution aqueuse :
- ayant une osmolalité entre 290 et 400 ;
- ayant un pH entre 6,0 et 8,0 ;
- éventuellement au moins un anti-coagulant choisi parmi l'EDTA, le citrate/acide citrique et/ou l'acide salicylique ; ou une combinaison de ceux-ci ;
et éventuellement contenant en outre un ou plusieurs composants pour les diluants connus par eux-mêmes,
c) lyser/dégarnir l'échantillon sanguin dilué de l'étape b) avec un agent de lyse qui est essentiellement exempt de cyanure et qui fournit, après la dilution à la dilution de WBC/Hb finale, un pH entre 4,0 et 7,5 et une concentration totale de composés d'ammonium quaternaire dans ladite dilution de WBC/Hb finale entre 1,5 et 4,5 grammes par litre ;
d) éventuellement compter, dans l'échantillon sanguin lysé/dégarni obtenu dans l'étape c), les leucocytes ou au moins une sous-population de ceux-ci, et/ou déterminer, dans l'échantillon sanguin lysé/dégarni dans l'étape c), la teneur en hémoglobine.

9. Procédé selon la revendication 8, dans lequel l'échantillon dans/où à l'étape d) les leucocytes et/ou la teneur en hémoglobine sont comptés/déterminés :
- a un pH entre 4,0 et 7,5 et pour ladite dilution de WBC/Hb finale ; et
- contient au moins un composé d'ammonium quaternaire à une concentration totale entre 1,5 et 4,5 grammes par litre.

10. Procédé selon la revendication 8 et/ou 9, dans lequel, dans l'étape d), au moins deux, et de préférence toutes les trois parmi les sous-populations de leucocytes composés des lymphocytes, de la « population de cellules mixte » et/ou des granulocytes sont comptés séparément.

11. Procédé selon l'une quelconque des revendications 8 à 10, dans lequel une partie de l'échantillon sanguin dilué obtenu dans l'étape b) est encore diluée, après quoi, dans ledit échantillon sanguin encore dilué, le dénombrement des globules rouges, le dénombrement des plaquettes et/ou le volume corpusculaire moyen sont déterminés.

12. Utilisation selon la revendication 1 ou 3, ou procédé selon l'une quelconque des revendications 8 à 11, dans laquelle le mammifère non humain est choisi parmi les animaux domestiques mammifères, comprenant, mais non limité à, les chats, les chiens et les chevaux ; et/ou de mammifères d'importance agronomique, comprenant mais non limités à, les moutons, les vaches/bétail, les chèvres, les agneaux et/ou les cochons.

13. Utilisation selon la revendication 1 ou 3, ou procédé selon l'une quelconque des revendications 8 à 11, dans laquelle le mammifère non humain est choisi parmi un chat, un chien et /ou un cheval.

14. Utilisation selon la revendication 1 ou 3, utilisation selon la revendication 12 ou 13 ou procédé selon l'une quelconque des revendications 8 à 11, dans laquelle l'analyse de l'échantillon sanguin est effectuée à l'aide d'une technique d'impédance d'ouverture.
